# EUROPEAN PATENT SPECIFICATION

(11) **EP 2 768 844 B1**
(45) Date of publication and mention of the grant of the patent: **27.04.2016**
(21) Application number: 11874171.9
(22) Date of filing: 21.10.2011
(51) Int. Cl.: C07K 1/20, C07K 1/18, C07K 14/56, C12N 15/21, C07K 1/16

(54) **SEPARATION OF ACETYLATED PROTEINS FROM UNACETYLATED PROTEINS**
TRENNUNG ACETYLIERTER PROTEINE VON NICHTACETYLIERTEN PROTEINEN
SÉPARATION DE PROTÉINES ACÉTYLÉES DE PROTÉINES NON ACÉTYLÉES

(43) Date of publication of application: 27.08.2014
(73) Proprietor: Tanvex Biologics Corp., Taipei (TW); La Jolla Biologics, Inc., San Diego, CA 92121 (US)
(72) Inventor: YU, Kuo-Ming, Taipei (TW); CHOU, Judy, Huei-chuan, Carlsbad, CA 92011 (US); HOPP, Jennifer, Renee, San Marcos, CA 92069 (US)
(74) Representative: 2K Patentanwälte Blasberg Kewitz & Reichel
(86) International application number: PCT/US2011/057244
(87) International publication number: WO 2013/058762

(56) References cited:
- EP-A2- 0 606 673
- WO-A1-94/20525
- WO-A1-94/20525
- WO-A1-2010/030222
- EVJENTH RUNE ET AL: "Application of reverse-phase HPLC to quantify oligopeptide acetylation eliminates interference from unspecific acetyl CoA hydrolysis", BMC PROCEEDINGS, BIOMED CENTRAL LTD, LONDON UK, vol. 3, no. Suppl 6, 4 August 2009 (2009-08-04), page S5, XP021060586, ISSN: 1753-6561, DOI: 10.1186/1753-6561-3-S6-S5
- NOGUEIRA R ET AL: "Alternative high-performance liquid chromatographic peptide separation and purification concept using a new mixed-mode reversed-phase/weak anion-exchange type stationary phase", JOURNAL OF CHROMATOGRAPHY, ELSEVIER SCIENCE PUBLISHERS B.V, NL, vol. 1089, no. 1-2, 30 September 2005 (2005-09-30), pages 158-169, XP027723871, ISSN: 0021-9673 [retrieved on 2005-09-30]
- BELDARRAIN, A. ET AL.: 'Chemical Characterization of RecombinanHtu man Leukocyte Interferon A Using Fast Atom Bombardment Mass Spectrometry' BIOTECHNOL. APPL. BIOCHEM. vol. 33, 2001, pages 173 - 182, XP055064585
- PADRON, G. ET AL.: 'Mass spectrometric analysis of recombinant human alpha-2 interferon' ANALYTICA CHIMICA ACTA vol. 223, 1989, pages 361 - 369, XP026724710
- TAKAO, T. ET AL.: 'Chemical Characterization of RecombinanHtu man Leukocyte Interferon A Using Fast Atom Bombardment Mass Spectrometry' J. BIOL. CHEM. vol. 262, no. 8, 1987, pages 3541 - 3547, XP055064586

## Description

### Field of the Invention

The invention relates to a process for separating acetylated proteins from unacetylated proteins. In particular, the invention relates to a process of using multimodal chromatography to separate acetylated proteins from unacetylated proteins.

### Background of the Invention

Modification of proteins extends the range of possible molecular structures beyond the limits imposed by the 20 encoded amino acids and, if reversible, gives a means of control and signaling. Acetylation occurs as a co-translational and post-translational modification of proteins. At least for eukaryotic proteins, acetylation is the most common covalent modification out of over 200 types that have been reported. Acetylation of proteins is catalyzed by a wide range of acetyltransferases that transfer acetyl groups from acetyl-coenzyme A to either the α-amino group of amino-terminal residues or to the ε-amino group of lysine residues at various positions. Amino-terminal acetylation occurs co-translationally on the bulk of acetylated eukaryotic proteins and post-translationally on prokaryotic ribosomal proteins and on processed eukaryotic regulatory peptides. Furthermore, ε-lysine acetylation occurs post-translationally on histones, high mobility group (HMG) proteins, transcription factors, nuclear receptors, and α-tubulin. Acetylation affects many protein functions, including enzymatic activity, stability, DNA binding, protein-protein interaction, and peptide-receptor recognition, and occurs on numerous and diverse proteins. Therefore, purification of acetylated protein is a subject in the art.

On the other hand, methods for producing recombinant proteins are well known in the art. Reports have shown recombinant protein derived from *E. coli* rendering a mixture of various forms due to post-translational modifications. For example, Susumu Honda *et al.* reported that *Escherichia coli*-derived human interferon-gamma with Cys-Tyr-Cys is partially acetylated at the N-terminus and suggests that the N-terminal portion of rIFN-7 is heterogeneous (Susumu Honda et al., Archives of Biochemistry and Biophysics, Vol. 269, No. 2, March, pp. 612-622, 1989*).* Elodie Charbaut *et al.* characterized five mammalian stathmin-like subdomains expressed in *Escherichia coli* by matrix-assisted laser desorption/ionization time-of-flight mass spectrometry and nanoESI-Q-TOF tandem mass spectrometry, and demonstrated that ectopic recombinant proteins can be extensively NK-acetylated in *E. coli,* and that the rules governing NK-acetylation are complex and involve the N-terminal region, as in eukaryotes. (Elodie Charbaut et al. FEBS Letters 529 (2002) 341-345). The isoforms of recombinant proteins include dimers, N-methionine-containing forms (US Patent No. 5,196,323), reduced forms, disulfide bridge isoforms (US Patent No. 4,765,903), and charge isoforms (Klein ML et al, Arch. Biochem. Biophys. ,276(2):531-7, 1990*).* Therefore, the separation of a correctly modified isoform is not simple, because these compositional and conformational variants have properties which are very similar to those of the native molecule.

Andreas O. Helbig *et al.* reported that by using dedicated mass spectrometry-based proteomics techniques it is possible to unravel N-terminal processing in a semi-comprehensive way. The reported multiprotease approach led to the identification of 1391 acetylated human protein N termini in HEK293 cells and revealed that the role of the penultimate position on the cleavage efficiency by the methionine aminopeptidases is essentially conserved from *Escherichia coli* to humans (Andreas O. Helbig et al., Molecular & Cellular Proteomics 9:928- 939, 2010). Moreover, a number of different chromatographic approaches have been used and combined for the separation of acetylated proteins. The most common protein separation methods are predicated on differences in the size, charge, and solubility between the protein to be purified and contaminants. Protocols based on these parameters include affinity chromatography, ion exchange chromatography, size exclusion chromatography, and hydrophobic interaction chromatography. Evert-Jan Sneekesa *et al.* disclose that capillary polystyrene-divinylbenzene (PS-DVB) monolithic columns were used to separate differentially acetylated intact IM9 protein isoforms and suggest that not only the number, but also the location of the acetylations on the protein had a significant effect on retention (Evert-Jan Sneekesa et al., Journal of Chromatography A, 1194 (2008) 199 - 204). US 6,620,918 provides a process for separating polypeptide monomers from dimers and/or other multimers using ion-exchange chromatography. US 6,005,075 demonstrates a process of purifying interferon-alpha-2a; in brief, chromatographic schemes have been demonstrated after refolding in 0.6 M guanidine solution and oxidation with air.

However, separation of acetylated proteins has not yet been achieved. Therefore, there still is a need to develop a process for separation of acetylated proteins with high recovery rate.

### Brief Description of the Drawing

Figure 1 shows the multimodal chromatogram of IFN-alpha-2a.
Figure 2 shows 14% SDS-PAGE with silver staining of IFN-alpha-2a. The assay was performed based on EP 6.0 monograph-interferon alpha-2a concentrated solution. M: marker; Lane 1: Roferon, 1µg; Lane 2: In-house Ref. IFN-alpha-2a, 0.05 µg; Lane 3: In-house Ref. IFN-alpha-2a, 0.25 µg; Lane 4: In-house Ref. IFN-alpha-2a, 1.25 µg; Lane 5: In-house Ref. IFN-alpha-2a, 6.25 µg; Lane 6: In-house Ref. IFN-alpha-2a, 31.25µg; Lane 7: Purified bulk of IFN-alpha-2a, 5 µg; Lane 8: Purified bulk of IFN-alpha-2a, 25 µg.
Figure 3 shows the multimodal chromatogram of IFN-alpha-2b.
Figure 4 shows 14% SDS-PAGE with silver staining of IFN-alpha-2b. The assay was performed based on EP 6.0 monograph-interferon alpha-2b concentrated solution. M: marker; Lane 1: In-house Ref. IFN-alpha-2b, 0.05 µg; Lane 2: In-house Ref. IFN-alpha-2b, 0.25 µg; Lane 3: In-house Ref. IFN-alpha-2b, 1.25 µg; Lane 4: In-house Ref. IFN-alpha-2b, 6.25 µg; Lane 5: In-house Ref. IFN-alpha-2b, 31.25 µg; Lane 6: Purified bulk IFN-alpha-2b, 5 µg; Lane 7: Purified bulk IFN-alpha-2b, 25 µg.
Figure 5 shows purity and related proteins by RP-HPLC. Samples were diluted to 1 mg/mL with water and injected 50 µg onto C18 column. The chromatographic condition was carried out based on EP 6.0 monograph-interferon alpha-2 concentrated solution.

### Detailed Description of the Invention

The invention successfully develops a simple and time-saving process employing multimodal chromatography to separate acetylated proteins from unacetylated proteins; for example, separation of desired acetylated proteins or removal of acetylated variants generated during microbial recombinant protein production.

The publications discussed herein are provided solely for their disclosure prior to the filing date of the present application.

Unless otherwise defined herein, scientific and technical terms used in connection with the present invention shall have the meanings that are commonly understood by those of ordinary skill in the art. The meaning and scope of the terms should be clear; however, in the event of any latent ambiguity, definitions provided herein take precedent over any dictionary or extrinsic definition.

As used herein and in the appended claims, the singular forms "a," "an," and "the" include plural reference unless the context clearly indicates otherwise.

The term "recombinant protein" as used herein defines a protein which one desires to recover in a relatively pure form and includes proteins having the amino acid sequence of native proteins and their analogs and muteins having substituted, deleted, replaced, or otherwise modified sequences.

The term "recombinant expression vector" refers to a plasmid or phage or virus or vector, for expressing a protein from a DNA (RNA) sequence. An expression vehicle can comprise a transcriptional unit comprising an assembly of (1) a genetic element or elements having a regulatory role in gene expression, for example, promoters or enhancers, (2) a structural or coding sequence which is transcribed into mRNA and translated into protein, and (3) appropriate transcription initiation and termination sequences.

The term "post-translational modification" means the chemical modification of a protein after its translation.

The term "co-translation" means the synthesis of a hybrid molecule consisting of the desired protein product covalently linked to a normal protein secreted by the organism being used, through translation of a hybrid mRNA molecule generated on the recombinant DNA.

The term "transformation" means introducing DNA into an organism so that the DNA is replicable, either as an extrachromosomal element, or by chromosomal integration. The term "transfection" refers to the taking up of an expression vector by a suitable host cell, whether or not any coding sequences are in fact expressed

The terms "biologically activity" and "biological activities" as used herein, refer to structural, regulatory, biochemical or other biological functions in living systems, for example similar or identical to naturally or non-naturally occurring molecules.

The term "multimodal chromatography support" refers to one capable of providing at least two different but co-operative sites which interact with the compound to be bound. For example, one of these sites may give an attractive type of charge-charge interaction between the ligand and the substance of interest. The other site may give electron acceptor-donor interaction and/or hydrophobic and/or hydrophilic interactions. Electron donor-acceptor interactions include interactions such as hydrogen-bonding, .pi.-.pi., cation-.pi., charge transfer, dipole-dipole, induced dipole etc. "Multimodal chromatography supports" are also known as "mixed mode" separation matrices. US 7,714,112 relates to a method of separating antibodies from other compound(s) in a liquid sample, wherein a mobile phase comprising said sample is contacted with a multimodal separation matrix to adsorb undesired compounds while the antibodies remain free in the liquid, wherein the multimodal separation matrix comprises first groups, which are capable of interacting with negatively charged sites of the target compounds, and second groups, which are capable of at least one interaction other than charge-charge interaction with said target compounds.

The term "chromatography" embraces a family of closely related separation methods. The feature distinguishing chromatography from most other physical and chemical methods of separation is that two mutually immiscible phases are brought into contact wherein one phase is stationary and the other mobile.

The term "functionality" is used herein for a group or a molecule which is functional in the sense that it is capable of interacting with a target molecule, or target compound.

The term "eluent" is used in its conventional meaning in this field, i.e. a buffer of suitable pH and/or ionic strength to release one or more compounds from a separation matrix.

The term "interferon" refers to a family of secreted proteins produced by a variety of eukaryotic cells upon exposure to various environmental stimuli, including virus infection or exposure to a mitogen. In addition to having anti-viral properties, interferons have been shown to affect a wide variety of cellular functions. All interferon units are expressed herein with reference to WHO international standards, 94/786 (rHuIFN-.alpha. consensus) and 95/650 (rHuIFN-.alpha.2a). According to the invention, the interferon includes consensus interferon and interferon-like product.

In one aspect, the invention provides a process for separating acetylated proteins from unacetylated proteins, comprising applying an impure preparation comprising acetylated and unacetylated proteins to a multimodal chromatography support and conducting elution with a change in pH.

According to the invention, an impure preparation is any involving acetylated proteins and unacetylated proteins. Preferably, the impure preparation is applied at a pH between 6 and 7. Preferably, the impure preparation is derived from cells. More preferably, the impure preparation is derived from prokaryotic cells.

According to the invention, the multimodal chromatography support is used for separation of acetylated proteins from unacetylated proteins. The multimodal chromatography support comprises hydrophobic groups and anion-exchanging groups.

The multimodal chromatography support is preferably provided in a chromatography column. Preferably, the multimodal chromatography support is Capto^{™} adhere. Multimodal chromatography is disclosed in Kjell Eriksson et al., BioProcess International 7(2) (February 2009); Jie Chen et al., 2010, Journal of Chromatography A, 1217, pp.216-224; and US Patent No. 7,714,112. In these prior art references, multimodal chromatography is used to isolate monoclonal antibodies. None of these references uses multimodal chromatography in the separation of acetylated proteins.

In an embodiment, multimodal chromatography support is used in liquid chromatography, i.e. by passing a mobile phase over a chromatography column comprising the multimodal chromatography support. The support may be in the form of porous or non-porous particles, such as essentially spherical particles, a monolith, filter, membrane, surface, capillaries, or any other commonly used format. In an alternative embodiment, the multimodal chromatography support is used in expanded bed chromatography i.e. by adding the mobile phase to an expanded bed of separation matrix in the form of particles, such as essentially spherical particles, comprising a high density filler. In another alternative embodiment, the multimodal chromatography support is used in a batch-wise process, wherein the separation matrix is added to a vessel comprising the liquid sample.

According to the invention, the hydrophobic groups of the multimodal chromatography support comprise aromatic groups, heteroaromatic or an non-aromatic hydrophobic groups (such as alkyl groups). Preferably, the hydrophobic group is hexyl group, butyl group, phenyl group or ether group.

According to the invention, the anion-exchanging groups of the multimodal chromatography support may be strong anion exchangers. In this context, the term "strong" anion exchangers is understood as groups which remain charged within a wide pH range. In an advantageous embodiment, the strong anion exchanging groups are quaternary amines. In an alternative embodiment, the anion-exchanging groups of the multimodal chromatography support may be weak ion exchangers. In this context, the term "weak" anion exchangers is understood to mean groups that are charged at certain pH values but may lose charge by a pH switch.

In one embodiment, for separating an acetylated protein, the sample is contacted with the multimodal chromatography support to adsorb the acetylated protein and an elution is subsequently conducted with a change in pH so that the unacetylated protein is eluted while retaining acetylated proteins on the support. In one embodiment, in the first step, a sample comprising the acetylated proteins is passed over a multimodal chromatography support under conditions allowing adsorption of the acetylated proteins to the support. Care should be taken not to exceed the capacity of the support, i.e. the flow should be sufficiently slow to allow a satisfactory adsorption. In a subsequent step, an elution is conducted using the multimodal chromatography support under a pH gradient and other conditions that elute unacetylated proteins. According to the invention, the change in pH for elution is step-wise or gradient. Preferably, the change in pH is carried out between 7 and 3. More preferably, protein mixture loaded in a pH range from 6.0 to 7.0 and the change in pH is carried out between 5.5 to 3.0. In a preferred embodiment, the elution solution is comprised of acetate ad ammonium. According to the invention, the other conditions that elute unacetylated proteins are commonly provided by the salt concentration i.e. ionic strength, hydrophobicity etc. The general principles of chromatography are well known in this field, and skilled persons in this field can easily adopt the necessary parameters for use of the present process.

In a preferred embodiment, if required, one or more washing steps may be applied before or between any such steps of the process of the invention.

According to the invention, the processes can be used in the separation of post-translational acetylated proteins and acetylated impurities generated during co-translation of a recombinant protein production in an expression system. Preferably, the processes of the invention can be used in the purification of insulin, somatotropins, interleukins, interferons or somatomedins.

In one embodiment, the process of the invention can be used in the purification of recombinant protein to remove acetylated forms therefrom. Accordingly, the invention provides a process of purifying a recombinant protein product obtained from an expression system, comprising i) applying a refolded recombinant protein mixture obtained from an expression system to a multimodal chromatography support and ii) conducting elution with a change in pH to obtain a recombinant protein product essentially free of acetylated proteins. Preferably, the expression system is a microbial, yeast or mammalian expression system.

In a further embodiment, the process of the invention is used to purify a recombinant interferon. The invention thus provides a process of purifying recombinant interferons produced from an expression system to obtain unacetylated forms thereof, comprising the steps of:
(a) subjecting solubilized and refolded interferons produced from an expression system to hydrophobic interaction chromatography, wherein the interferons comprise acetylated forms and unacetylated forms; and
(b) subjecting the interferons from step (a) to the process for separating acetylated proteins from unacetylated proteins mentioned herein so that the unacetylated form interferons can be obtained.

According to the invention, in an expression system, the interferons to be purified can be obtained from host cells, such as *Escherichia coli* or *Saccharomyces cerevisiae,* transformed with an expression vector containing the gene encoding the acetylated protein as disclosed in prior art. More preferably, the interferon is interferon-alpha. More preferably, the interferon is IFN-α2a and □ IFN-α2b. The recombinant host cells are recovered from the culture medium by means of centrifugation or filtration, and then broken applying conventional techniques, e.g. osmotic shock, sonication, milling or chemical agents, in order to isolate the insoluble bodies. The insoluble bodies are preferably washed by a buffer, preferably, Tris buffer. In one embodiment, the inclusion bodies are washed with Tris/guanidine hydrochloride (GdHCl) buffer.

According to the process of the invention, in step (a), the solubilized and refolded interferons produced from an expression system are subjected to hydrophobic interaction chromatography (HIC). HIC is a powerful technique for both analytical and preparative separations of biomolecules. The technique takes advantage of the hydrophobic areas located on the surface of proteins. HIC is suitable in difficult separations, particularly those in which the impurities are of similar isoelectric point or molecular weight. Chromatographic columns suitable for the purpose may be of various sections and lengths and HIC resins are selected from those available commercially. The pH during the contact stage between the protein and the adsorbent material is that of the solution supplied. According to the process of the invention, suitable HIC resin materials for the purpose are selected from those which can bind the interferon hydrophobically and which are available commercially under various trade marks. Preferable, the HIC column is Toyopearl series (Tosch Biosience LLC). More preferably, the HIC column is Toyopearl Buty-650M.

According to the process of the invention, in step (b), the interferons from step (a) are further subjected to the process of the invention employing a multimodal chromatography. The multimodal chromatography is preferably based on a rigid agarose matrix that allows high fluid velocities to be used. The high cross-linked agarose base support gives the medium high chemical and physical stability. The multimodal chromatography support comprises first groups, which are capable of interacting with negatively charged sites of the target compounds, and second groups, which are capable of at least one interaction other than charge-charge interaction with said target compounds. The multimodal chromatography support is provided in a chromatography column. Preferably, the multimodal chromatography uses Capto^{™} adhere.

According to the invention, HIC and multimodal chromatography are capable of separating the sequence variant. The first column step of HIC aims to remove covalent aggregates and the second multimodal chromatography column is intended to separate acetylated variants.

According to the invention, the solubilized and refolded interferons in step (a) are obtained by solubilizing the insoluble bodies of the recombinant interferons obtained from a recombinant protein production in an expression system and then refolding the resulting interferons in the presence of an oxidation reagent. According to the invention, any suitable agent capable of solubilizing insoluble bodies can be used in the invention. In one embodiment of the invention, a chaotrophic agent or a detergent can be used for solubilization of inclusion bodies. Preferably, the chaotrophic agent is urea, guanidine hydrochloride and the detergent is SDS, N-acetyl trimethyl ammonium chloride or sodium N-lauroyl sarcosine. More preferably, the solubilization agent is guanidine hydrochloride. Techniques of separation and solubilization of insoluble bodies are known in the art, such as those disclosed in Journal of Bioscience and Bioengineering, Vol. 99, No.4, 303-310, 2005. In one embodiment, the inclusion bodies are dissolved in Tris/GdHCl/Tween to obtain interferon at 1-5 mg/mL.

According to the invention, refolding of solubilized interferons is performed in the presence of an oxidation reagent. The oxidation reagent can oxidize cysteines selectively. Preferably, the oxidation reagent is iodine or iodosobenzoate; preferably, the oxidation reagent is o-iodosobenzoate. The oxidation reagent is used to oxidize cysteine residues selectively and stoichiometrically. In this regard, the term "selectively" indicates that the oxidation reagent (1) oxidizes the cysteines to the disulfide level with no or insignificant oxidation to higher levels and (2) preferentially oxidizes active cysteines that are positioned proximately in the reduced protein. The mole ratio of oxidation reagent to synthetic protein may vary widely depending on the oxidation reagent used. The mole ratio will be at least stoichiometric (1:1 or greater) and will typically be in the range of 1:1 to 100:1. In the case of o-iodosobenzoate, the mole ratio will usually be in the range of about 1:1 to about 5:1. In all instances, the oxidation reagent is in excess during the terminal portion of the reaction to ensure complete oxidation of the reduced protein. These conditions may be achieved by running the reaction with excess oxidation reagent over its entire duration or running the reaction with approximately equimolar portions of reactants over the majority of the reaction period and adding excess oxidant near the end of the reaction period. Oxidation of the thiol group such as by using o-iodosobenzoate can be referenced in US Patent No. 4,530,787.

Removing host impurities while maintaining high recovery is a major objective of this invention. The invention demonstrates a process employing a two-column step, instead of the four-column step in chromatography in the art. The product obtained herein has high purity and yield; for example, up to 30-40% of overall recovery and yield higher than 95% purity can be achieved.

### Examples

### Example 1 Purification of IFN-alpha-2a Using the Process of the Invention Gene cloning, plasmid construct and cell culturing

The IFN-alpha gene cloning, plasmid constructs and codon optimization were performed according to recombinant DNA technology. Recombinant IFN-alpha-2a was expressed as insoluble inclusion bodies in bacteria BL21 (DE3) cells with plasmid PET30a containing the gene of IFN-alpha-2a. Fermentation was carried out with a dissolved oxygen level set at 30% in a 5-L fed-batch fermentation. After three hours of induction at 1mM IPTG, cells were harvested from the broth by centrifugation. Cell paste was washed with 20 mM Tris pH 7.4/ 0.5 mM EDTA buffer and then resuspended in the same buffer solution for cell breaking-down by ultrasonication. IB was collected by centrifugation.

### Inclusion body solubilization

IB was dissolved with appropriate volume of 50 mM Tris pH7.4/7.0 GdHCl/2% tween 80 to obtain IFN-alpha-2a at 4 mg/mL. Complete dissolution was achieved by gentle stirring at room temperature for 1 hr. The protein solution was diluted 11-fold with 50 mM Tris pH7.4 buffer. Insoluble debris was removed by cold centrifuge for 30 min.

### Disulfide oxidation

1 mM iodobenoate in 50 mM Tris pH7.4 buffer was prepared prior to disulfide oxidation. 10 µM iodobenoate was introduced into protein solution five times every hour (total 50 µM). The protein solution was incubated at room temperature with gentle stirring.

### Hydrophobic interaction chromatography

1 mM EDTA and 10 mM methionine were added into the protein solution, followed by addition of solid ammonium sulfate to obtain 1 M. The pH was adjusted to 6.5 with acetic acid. After removing particulates by centrifuge and filtration, chromatographic purification was carried out as follows:
Toyopearl Butyl-650M, 5x5 cm, flow rate 25 ml/min.
Buffer A: 20 mM NaPi pH 6.5/1.0M AmSO₄/1 mM EDTA/10 mM methionine;
Buffer B: 20 mM NaPi pH 6.5/1 mM EDTA/10 mM methionine.

The protein solution was loaded onto the column equilibrated with Buffer A. After loading, the column was washed with at least 5 CV of 100% Buffer A until OD280 reaching near zero. Protein was eluted with a series of steps: 20% B over 2 CV, 40% B over 2 CV, 60% B over 2 CV, 80% B over 2 CV and 100% B over 2 CV. The column was cleaned with water, 0.5N NaOH and 0.1N HCl, followed by regenerating with Buffer A. Each fraction of 18 mL was collected and samples were taken for analysis by RP-HPLC. Fractions essentially free from aggregated IFN-alpha-2a were pooled.

### Multimodal chromatography

5 mM ammonium acetate pH 6.5 buffer was introduced to dilute ammonium sulfate until a final conductivity less than 15 mS/cm. Chromatographic purification was carried out as follows:
Capto Adhere 2.6x6 cm, flow rate 6.7 ml/min.
Buffer C: 25 mM ammonium acetate buffer pH 6.5 by adding 50% acetic acid;
Buffer D: 5 mM acetic acid.

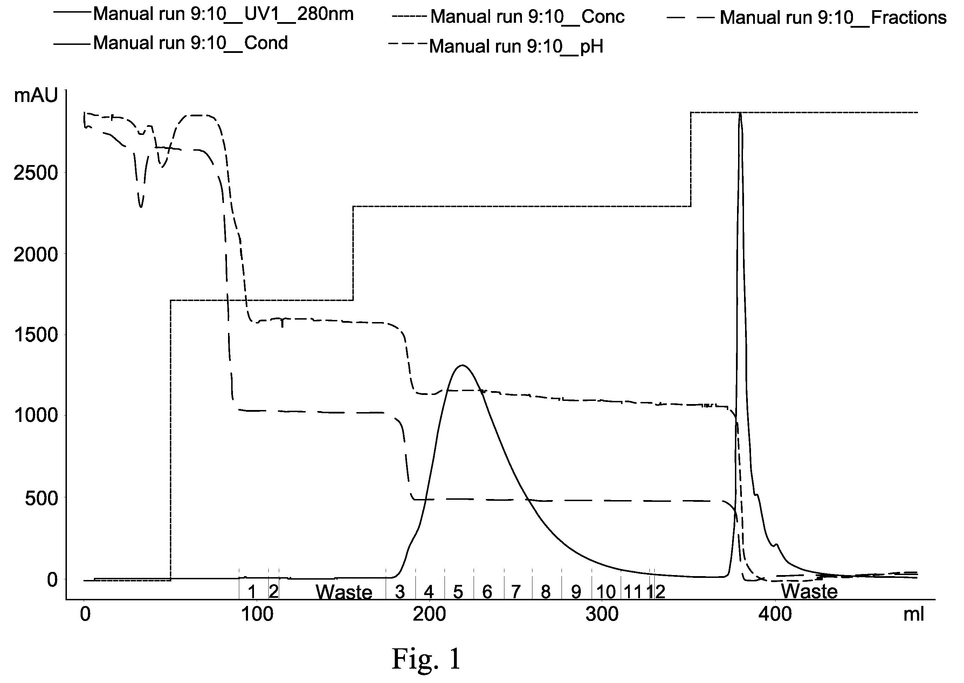

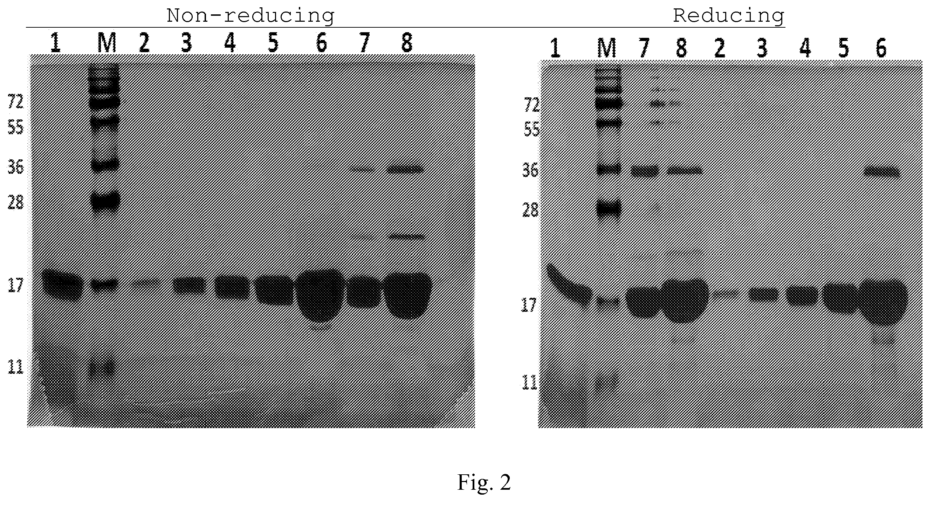

The diluted protein solution was loaded onto the column previously equilibrated with Buffer C. When completed, the column was washed with 3 CV of 60% D. The target protein was eluted with 80% D. The chromatogram of IFN-alpha-2a is shown in Fig. 1. The column was cleaned with 100% C followed by 2M NaCl, 1N NaOH, 2M NaCl and 0.1M acetic acid. Each fraction of 17-mL was collected and samples were taken for analysis by RP-HPLC. Fractions essentially free from acetylated isoforms were pooled. The resulting product was evaluated by comparing respective purity. The results of SDS-PAGE analysis are as shown in Fig. 2.

### Sterile filtration

Fractions from Capto Adhere column meeting specifications in purity were pooled and concentrated to over 3 mg/mL using 3-KD centrifugal filters. The purified bulk was further filtered through a 0.2 µm membrane. Each step yield of IFN-alpha-2a is shown in Table I. The amount of the acetylated IFN was about 20-30% in inclusion bodies. After performing the above-mentioned chromatographic steps, the acetylated IFN is not detectable.

**Table I. Sample name: IFN-alpha-2a**

| Step of process | Product (mg) | Yield (%) | Purity by RP-HPLC (%) |
|---|---|---|---|
| Crude IB | 333 (native 250) | N/A | approx. 40 |
| IBA oxidation | 137.9 | 55.2 | 46.5 |
| Butyl-650M | 116.2 | 84.3 | 75.9 |
| Capto Ahere | 80.2 | 69.0 | 96.6 |
| Conc./filtration | 73.9 | 92.1 | 97.4 |
| Overall yield: 29.6% | | | |

### Example 2 Purification of IFN-alpha-2b using the process of the invention

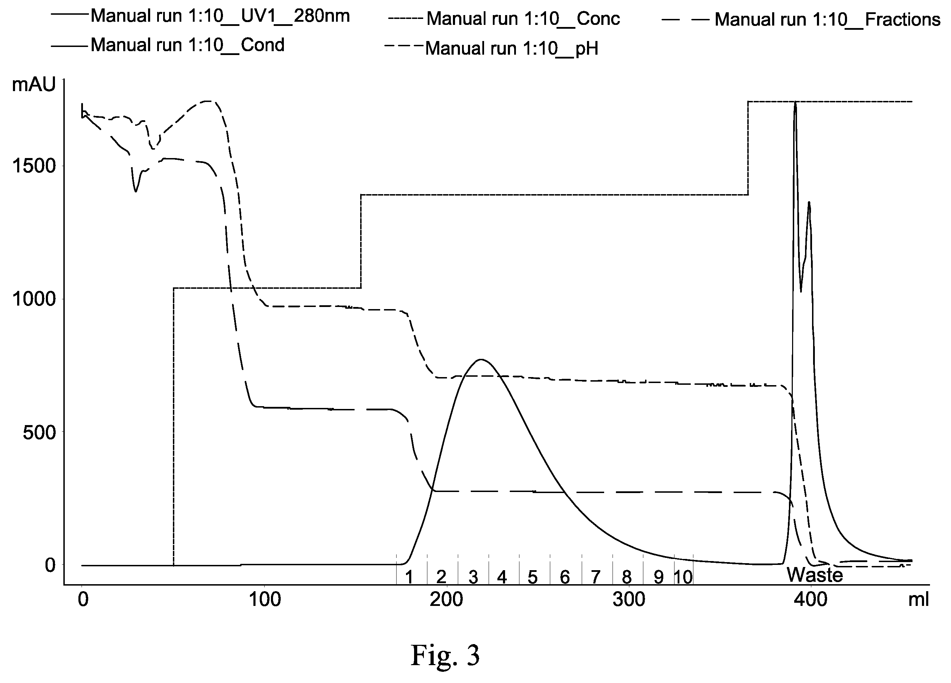

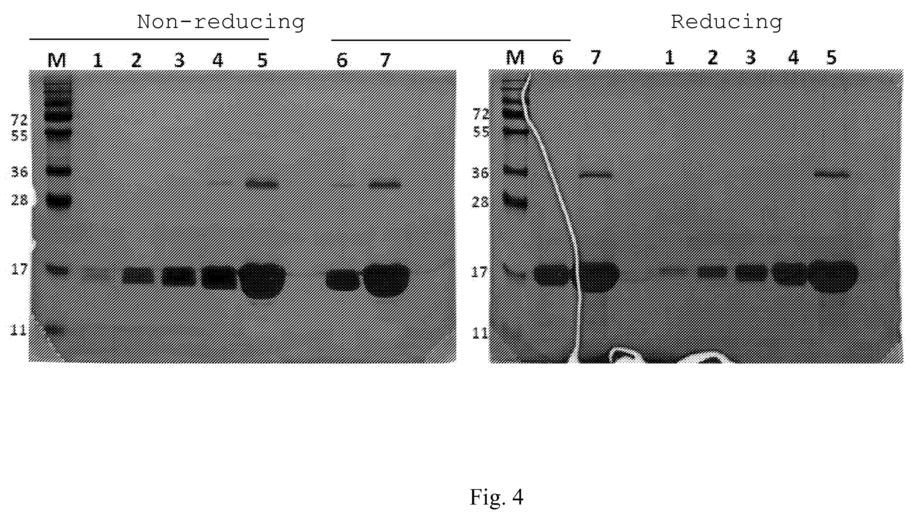

The production of IFN-alpha-2b was carried out in the same manner as Example 1. The overall yield of IFN-alpha-2b is shown in Table II. The resulting fractions essentially free from acetylated isoforms were pooled. The chromatogram of IFN-alpha-2b is shown in Fig. 3. The resulting product was evaluated by comparing respective purity. The results of SDS-PAGE analysis are as shown in Fig. 4. The amount of the acetylated IFN was about 20-30% in inclusion bodies. After performing the above-mentioned chromatographic steps, the acetylated IFN is not detectable.

**Table II. Sample name: IFN-alpha-2b**

| Step of process | Product (mg) | Yield (%) | Purity by RP-HPLC (%) |
|---|---|---|---|
| Crude IB | 349 (native 262) | N/A | approx. 40 |
| IBA oxidation | 202.7 | 77.4 | 45.3 |
| Butyl-650M | 158.3 | 78.1 | 69.8 |
| Capto Ahere | 111.3 | 70.3 | 97.7 |
| Conc./filtration | 110.7 | 99.5 | 97.3 |
| | Overall yield: | 42.2% | |

### Example 3 Analytical Tests of IFN-alpha-2a of Example 1 and IFN-alpha-2b of Example 2

The IFN-alpha-2a of Example 1 and IFN-alpha-2b of Example 2 were analyzed.

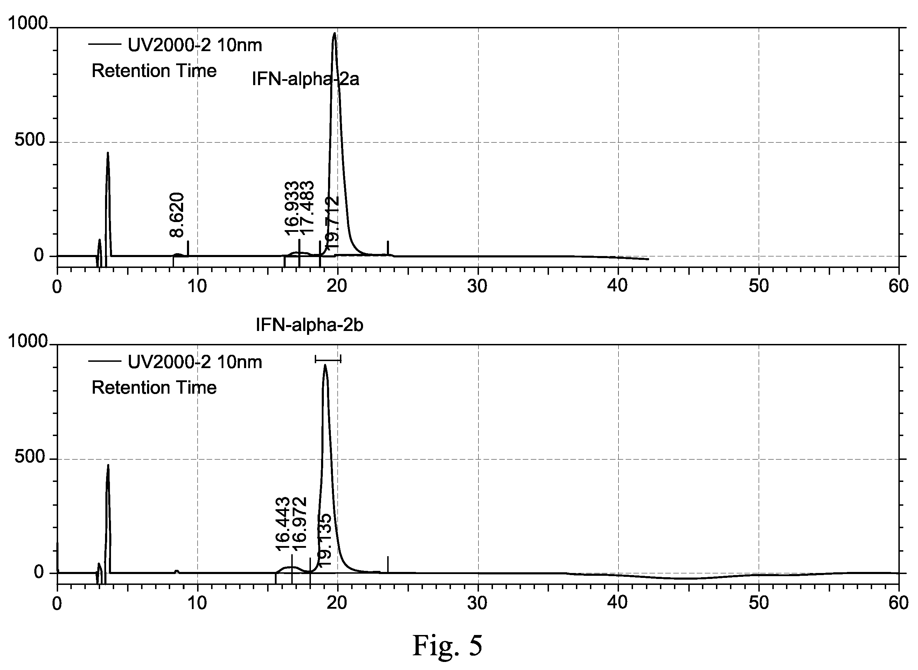

The purities of IFN-alpha-2a and IFN-alpha-2b and related protein impurities determined by RP-HPLC are shown in Fig. 5.

## Claims

1. A process for separating acetylated proteins from unacetylated proteins, comprising applying an impure preparation comprising acetylated and unacetylated proteins to a multimodal chromatography support and conducting elution with a change in pH, wherein the multimodal chromatography support comprises hydrophobic groups and anion-exchanging groups.

2. The process of Claim 1, wherein the hydrophobic groups of the multimodal chromatography support comprise aromatic groups, heteroaromatic or an non-aromatic hydrophobic groups (such as alkyl groups).

3. The process of Claim 1 or 2, wherein the hydrophobic group is a hexyl group, butyl group, phenyl group or ether group.

4. The process of any of the preceding claims, wherein the anion-exchanging groups of the multimodal chromatography support may be strong anion exchangers.

5. The process of any of the preceding claims, wherein the multimodal chromatography support is Capto^{™} adhere.

6. The process of any of the preceding claims, wherein the impure preparation is applied at a pH between 6 and 7.

7. The process of any of the preceding claims, wherein the impure preparation is derived from cells.

8. The process of any of claims 1 to 6, wherein the impure preparation is derived from prokaryotic cells.

9. The process of any of the preceding claims, wherein the change in pH for elution is step-wise or gradient.

10. The process of any of the preceding claims, wherein the change in pH is carried out between 7 and 3.

11. The process of any of claims 1 to 9, wherein the change in pH of elution is carried out between 5.5 to 3.0.

12. The process of any of the preceding claims, wherein the elution is conducted with a solution comprising acetate and ammonium.

13. The processes of any of the preceding claims, which can be used in the purification of insulin, somatotropins, interleukins, interferons or somatomedins.

## Patentansprüche

1. Verfahren zur Trennung von acetylierten Proteinen von aus nichtacetylierten Proteinen, umfassend
Aufbringen einer unreinen Zubereitung mit acetylierten und nicht-acetylierten Proteinen auf einen multimodalen Chromatographieträger und Durchführen einer Auswaschung mit einer Änderung des pH-Wertes, wobei der multimodale Chromatographieträger hydrophoben Gruppen und Anionen austauschende Gruppen umfasst.

2. Verfahren nach Anspruch 1, wobei die hydrophoben Gruppen des multimodalen Chromatographieträgers aromatische Gruppen, heteroaromatische Gruppen oder eine nicht-aromatische hydrophobe Gruppe (beispielsweise Alkylgruppen) umfassen.

3. Verfahren nach Anspruch 1 oder 2, wobei die hydrophobe Gruppe ist eine Hexyl-Gruppe, Butyl-Gruppe, Phenyl-Gruppe oder Ether-Gruppe ist.

4. Verfahren nach einem der vorhergehenden Ansprüche, wobei die Anionen austauschenden Gruppen des multimodalen Chromatographieträgers starke Anionenaustauscher sein können.

5. Verfahren nach einem der vorhergehenden Ansprüche, wobei der multimodale Chromatographieträger ein Capto^{™}-Kleber ist.

6. Verfahren nach einem der vorhergehenden Ansprüche, wobei die unreine Zubereitung bei einem pH-Wert zwischen 6 und 7 aufgebracht wird.

7. Verfahren nach einem der vorhergehenden Ansprüche, wobei die unreine Zubereitung aus Zellen abgeleitet wird.

8. Verfahren nach einem der Ansprüche 1 bis 6, wobei die unreine Zubereitung aus prokaryotischen Zellen abgeleitet wird.

9. Verfahren nach einem der vorhergehenden Ansprüche, wobei die Änderung des pH-Wertes für die Auswaschung stufenweise oder entsprechend einem Gradienten erfolgt.

10. Verfahren nach einem der vorhergehenden Ansprüche, wobei die Änderung des pH-Wertes zwischen 7 und 3 durchgeführt wird.

11. Verfahren nach einem der Ansprüche 1 bis 9, wobei die Änderung des pH-Wertes der Auswaschung zwischen 5,5 bis 3,0 durchgeführt wird.

12. Verfahren nach einem der vorhergehenden Ansprüche, wobei die Auswaschung mit einer Lösung durchgeführt wird, die Acetat und Ammonium enthält.

13. Verfahren nach einem der vorhergehenden Ansprüche, die bei der Reinigung von Insulin, Somatotropinen, Interleukinen, Interferonen oder Somatomedinen verwendet werden kann.

## Revendications

1. Un procédé de séparation de protéines acétylées de protéines non acétylées, comprenant l'application d'une préparation impure comprenant des protéines acétylées et non acétylées à un support de chromatographie multimodal et la mise en oeuvre d'une élution avec modification du pH, dans lequel le support de chromotographie multimodal comporte des groupes hydrophobes et des groupes échangeurs d'anions.

2. Le procédé selon la revendication 1, dans lequel les groupes hydrophobes du support de chromatographie multimodal comportent des groupes aromatiques, hétéro-aromatiques ou un groupe hydrophobe non-aromatique (tels que des groupes alkyle).

3. Le procédé selon la revendication 1 ou 2, dans lequel le groupe hydrophobe est un groupe hexyle, un groupe butyle, un groupe phényle ou un groupe éther.

4. Le procédé selon l'une quelconque des revendications précédentes, dans lequel les groupes échangeurs d'anions du support de chromatographie multimodal peuvent être des échangeurs d'anions forts.

5. Le procédé selon l'une quelconque des revendications précédentes, dans lequel le support de chromatographie multimodal est adhérent Capto™

6. Le procédé selon l'une quelconque des revendications précédentes, dans lequel la préparation impure est appliquée à un pH compris entre 6 et 7.

7. Le procédé selon l'une quelconque des revendications précédentes, dans lequel la préparation impure est dérivée de cellules.

8. Le procédé selon l'une quelconque des revendications précédentes, dans lequel la préparation impure est dérivée à partir de cellules procaryotes.

9. Le procédé selon l'une quelconque des revendications précédentes, dans lequel le changement de pH pour l'élution est par pas progressif ou par gradient.

10. Le procédé selon l'une quelconque des revendications précédentes, dans lequel le changement de pH est effectué entre 7 et 3.

11. Le procédé selon l'une quelconque des revendications 1 à 9, dans lequel la modification du pH d'élution est effectuée entre 5 5. à 3,0.

12. Le procédé selon l'une quelconque des revendications précédentes, dans lequel l'élution est effectuée avec une solution comprenant de l'acétate et de l'ammonium.

13. Le procédé de l'une quelconque des revendications précédentes, pouvant servir à la purification de l'insuline, des somatotropines, des interleukines, des interférons ou des somatomédines.
